(19) Europäisches Patentamt / European Patent Office / Office européen des brevets

(11) **EP 3 943 095 A1**

(12) **EUROPEAN PATENT APPLICATION**
published in accordance with Art. 153(4) EPC

(43) Date of publication:
**26.01.2022 Bulletin 2022/04**

(21) Application number: **20774006.9**

(22) Date of filing: **03.03.2020**

(51) International Patent Classification (IPC):
**A61K 36/896** (2006.01)    **A61K 36/886** (2006.01)
**A61P 1/10** (2006.01)

(52) Cooperative Patent Classification (CPC):
**A61K 36/886; A61K 36/896; A61P 1/10**

(86) International application number:
**PCT/CN2020/077631**

(87) International publication number:
**WO 2020/187019 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.03.2019 CN 201910216933**

(71) Applicant: **Tsing Hua De Ren Xi'an Happiness Pharmaceutical Co., Ltd.**
**Xi'an, Shaanxi 710043 (CN)**

(72) Inventor: **DU, Chengqiang**
**Shaanxi 710043 (CN)**

(74) Representative: **HGF**
**HGF Limited**
**1 City Walk**
**Leeds LS11 9DX (GB)**

(54) **TRADITIONAL CHINESE MEDICINE LAXATIVE COMPOSITION FOR TREATING CONSTIPATION, PREPARATION METHOD THEREFOR AND APPLICATION THEREOF**

(57) The present disclosure discloses a traditional Chinese medicine composition for loosening bowel to relieve constipation as well as a preparation method and an application thereof. The traditional Chinese medicine composition is prepared by extracting aloe and fructus aurantii with a mass ratio of 2-5:1-10.

EP 3 943 095 A1

**Description**

**Technical Field**

The present disclosure relates to the technical field of traditional Chinese medicines, in particular to a traditional Chinese medicine composition for loosening bowel to relieve constipation as well as a preparation method and an application thereof.

**Background**

Constipation is a common clinical symptom and mainly refers to reduction in frequency of defecation, reduction in quantity of faeces, dry stool, defecation exertion and the like. Symptomatic constipation can be diagnosed if two or more of the above symptoms exist at the same time. Generally, focusing on reduction in frequency of defecation, a person is diagnosed to have constipation if having a bowel movement once in 2-3 days or more days (or less than 3 times in each week) generally.

Great changes have taken place in modern people's lifestyle. Many people sit still for a long time, don't object to the finest food, and intake less and less cellulose, and some people drink too much. Various reasons cause digestion dysfunction, and constipation in large intestine is the most common phenomenon, which brings many persons unutterable agonies and influences physical health.

At present, there are many traditional Chinese medicine compositions for loosening bowel to relieve constipation on the market; however, the drugs are more in ingredients and complex in compositions generally, as the saying goes, all medicines have toxicity to some degree, and thus the risk of producing the side effect after the drugs are taken is increased to some extent.

**Summary**

The present disclosure aims to provide a traditional Chinese medicine composition for loosening bowel to relieve constipation as well as a preparation method and an application thereof so as to lower the risk of producing the side effect after the traditional Chinese medicine composition is taken.

In order to achieve the objective, according to one aspect of the present disclosure, the traditional Chinese medicine composition for loosening bowel to relieve constipation is provided. Active ingredients of the traditional Chinese medicine composition are prepared from extracts of raw materials which consist of aloe and fructus aurantii with a mass ratio of 2-5:1-10.

Furthermore, raw materials consist of the aloe and the fructus aurantii with a mass ratio of 5:7.

Furthermore, the traditional Chinese medicine composition further contains a pharmaceutically acceptable adjuvant.

Furthermore, the traditional Chinese medicine composition is in the following dosage forms: tablets, granules, capsules, pills, suppositories, powders, concentrated decoction, drops, aerosol, powder for inhalation, solution, a suspension, syrup, mixture, medicinal wine, medicinal tea, buccal tablets, freeze-dried powder injection or an emulsion.

Furthermore, the traditional Chinese medicine composition is prepared by the following steps of: S1, putting the aloe and the fructus aurantii with the mass ratio of 2-5:1-10 in an extracting tank for extraction with water, and obtaining an extracted fluid after extraction is finished, wherein steam pressure of extraction is 0.25-0.35MPa, and a temperature is 70-90°C; S2, performing vacuum concentration on the extracted fluid to obtain a extract, wherein a vacuum degree of vacuum concentration is -0.08 to -0.06MPa, steam pressure of extraction is 0.25-0.35MPa, and a temperature is 60-70°C; and S3, preparing the traditional Chinese medicine composition for loosening bowel to relieve constipation with the extract as the active ingredient.

Furthermore, extraction is performed two or more times.

Furthermore, extraction includes: adding 6-10 times water for the first time, and performing extraction for 4h; adding 4-8 times water for the second time, and performing extraction for 3h; and combining a first extracted fluid obtained by the first extraction with a second extracted fluid obtained by the second extraction, and filtering a mixture to obtain the extracted fluid.

Furthermore, S3 further includes the following steps of drying and crushing the extract to obtain dry paste powder, and then preparing the traditional Chinese medicine composition for loosening bowel to relieve constipation with the dry paste powder as the active ingredient.

According to another aspect of the present disclosure, a preparation method of the traditional Chinese medicine composition is provided. The preparation method comprises the following steps of: S1, putting the aloe and the fructus aurantii with the mass ratio of 2-5:1-10 in an extracting tank for extraction with water, and obtaining an extracted fluid after extraction is finished, wherein the steam pressure of extraction is 0.25-0.35MPa, and the temperature is 70-90°C;

S2, performing vacuum concentration on the extracted fluid to obtain a extract, wherein the vacuum degree of vacuum concentration is -0.08 to -0.06MPa, the steam pressure of extraction is 0.25-0.35MPa, and the temperature is 60-70°C; and S3, preparing the traditional Chinese medicine composition for loosening bowel to relieve constipation with the extract as the active ingredient. Preferably, extraction is performed two or more times. More preferably, extraction includes: adding 6-10 times water for the first time, and performing extraction for 4h; adding 4-8 times water for the second time, and performing extraction for 3h; and combining the first extracted fluid obtained by the first extraction with the second extracted fluid obtained by the second extraction, and filtering the mixture to obtain the extracted fluid. Further preferably, S3 further includes the following steps of drying and crushing the extract to obtain the dry paste powder, and then preparing the traditional Chinese medicine composition for loosening bowel to relieve constipation with the dry paste powder as the active ingredient.

According to another aspect of the present disclosure, use the traditional Chinese medicine composition for loosening bowel to relieve constipation in preparing a drug for loosening bowel to relieve constipation is provided.

By applying the technical solution of the present disclosure, the compositions of the traditional Chinese medicine composition for loosening bowel to relieve constipation are simple, and active ingredients are extracted from the aloe and the fructus aurantii, such that the situation that excessive reaction may occur among the complex compositions is avoided, the risk of producing the side effect after the traditional Chinese medicine composition is taken is obviously lowered, and the traditional Chinese medicine composition has good efficacy of loosening bowel to relieve constipation.

**Detailed Description of the Embodiments**

It should be noted that the embodiments of the present application and the characteristics in the embodiments can be combined with other another without conflict. The present disclosure will be described below in detail in combination with the embodiments.

The existing traditional Chinese medicine compositions for loosening bowel to relieve constipation on the market are more in ingredients and complex in compositions generally, and thus the risk of producing the side effect after the traditional Chinese medicine compositions are taken is increased to some extent. To lower the risk, the inventor of the present disclosure makes deep study on the traditional Chinese medicine composition for loosening bowel to relieve constipation. It is found accidentally in the study process that good effect can be achieved by mixing the aloe with the fructus aurantii according to a specific ratio as the extracts of the raw materials only, such that the situation that excessive reaction may occur among the complex compositions is avoided, the risk of producing the side effect after the drugs are taken is obviously lowered, and the unexpected effect is obtained.

According to a typical embodiment of the present disclosure, a traditional Chinese medicine composition for loosening bowel to relieve constipation is provided. The traditional Chinese medicine composition is prepared from extracts of raw materials which consist of the aloe and the fructus aurantii with the mass ratio of 2-5:1-10.

Considering the safety problem of an aloe-fructus aurantii composition, preferably, the raw materials consist of the aloe and the fructus aurantii with the mass ratio of 5:7.

According to a typical embodiment of the present disclosure, the traditional Chinese medicine composition further contains a pharmaceutically acceptable adjuvant and is in the following dosage forms: tablets, granules, capsules, pills, suppositories, powders, concentrated decoction, drops, aerosol, powder for inhalation, solution, a suspension, syrup, mixture, medicinal wine, medicinal tea, buccal tablets, freeze-dried powder injection or an emulsion. The traditional Chinese medicine composition may be a normal preparation, a sustained release preparation, a controlled release preparation and various particulate delivery systems.

The traditional Chinese medicine composition, containing an effective dose, of the present disclosure may be prepared by utilizing a drug carrier familiar to those skilled in the art, e.g., an oral preparation (such as tablets, capsules, solution or suspension) and an injectable preparation (such as an injectable solution or suspension, or injectable dry powder which can be immediately used with injection water before being injected). A carrier in a drug composition includes: an adhesive (such as starch, generally, corn starch, wheat starch or rice starch, gelatin, methylcellulose, sodium carboxymethylcellulose and/or polyvinylpyrrolidone), a diluent (such as lactose, dextrose, sucrose, mannitol, sorbitol, cellulose and/or glycerin), a lubricant (such as silicon dioxide, talcum, stearic acid or salt thereof, generally, magnesium stearate or calcium stearate and/or polyethylene glycol) as well as further a disintegrant (such as starch, agar, alginic acid or salt thereof, generally, sodium alginate) and/or an effervescent mixture, a cosolvent, a stabilizer, a suspending agent, a non-pigmented agent, a corrigent and the like as needed which are used by the oral preparation; a preservative, a solubilizer, a stabilizer and the like which are used by the injectable preparation; and a matrix, the diluent, the lubricant, the preservative and the like which are used by a local preparation. A pharmaceutic preparation may be administered orally or parenterally (for example, intravenously, subcutaneously, intraperitoneally or locally), and some drugs may be prepared into enteric-coated tablets if being unstable under the stomach conditions.

A term "effective dose" of the traditional Chinese medicine composition of the present disclosure refers to a sufficient amount of a compound suitable for reasonable benefit/risk ratio treatment obstacle of any medical treatment and/or prevention. However, it should be appreciated that a total daily dose of the traditional Chinese medicine composition of the present disclosure should be decided by an attending doctor in a reliable medical judgment range. For any specific patient, a specific treatment effective dose level should be determined according to various factors, and all the factors include an treatment obstacle and a severity level of the obstacle; the activity of an adopted specific compound; an adopted specific composition; an age, a weight, a general health status, a sex and a diet of the patient; an administration time, an administration route and an excretion rate of the adopted specific compound; a duration of treatment; a drug used in combination with or concurrently with the adopted specific compound; and similar factors well known in the medical field. For example, a practice in the art is as follows: the dose of the traditional Chinese medicine composition is gradually increased starting from a level lower than that required for the obtained needed treatment effect until the needed effect is obtained. In general, the dose of the traditional Chinese medicine composition of the present disclosure is used for mammals, particularly, humans, and may be between 3.6mg/kg·D and 109mg/kg·D (by 60kg for adults).

According to a typical embodiment of the present disclosure, the traditional Chinese medicine composition is prepared by the following steps of: S1, putting aloe and fructus aurantii with the mass ratio of 2-5:1-10 in an extracting tank for extraction with water, and obtaining an extracted fluid after extraction is finished, wherein steam pressure of extraction is 0.25-0.35MPa, and a temperature is 70-90°C; S2, performing vacuum concentration on the extracted fluid to obtain a extract, wherein a vacuum degree of vacuum concentration is -0.08 to -0.06MPa, steam pressure of extraction is 0.25-0.35MPa, and a temperature is 60-70°C; and S3, preparing the traditional Chinese medicine composition for loosening bowel to relieve constipation with the extract as the active ingredient.

Although all the compositions and contents thereof in the extract obtained by the steps cannot be specified, the inventor discovers that the traditional Chinese medicine composition obtained by the steps has relatively good curative effect, and meanwhile, the risk capable of producing the side effect is greatly lowered due to simple components of original raw materials.

In order to fully extracting the active ingredients, preferably, extraction is performed two or more times. More preferably, extraction includes: adding 6-10 times water for the first time, and performing extraction for 4h; adding 4-8 times water for the second time, and performing extraction for 3h; and combining a first extracted fluid obtained by the first extraction with a second extracted fluid obtained by the second extraction, and filtering a mixture to obtain the extracted fluid.

According to a typical embodiment of the present disclosure, S3 further includes the following steps of drying and crushing the extract to obtain dry paste powder, and then preparing the traditional Chinese medicine composition for loosening bowel to relieve constipation with the dry paste powder as the active ingredient. Therefore, storage and industrial production of the active ingredients are more convenient.

According to a typical embodiment of the present disclosure, a preparation method of the traditional Chinese medicine composition is provided. The preparation method comprises the following steps of: S1, putting the aloe and the fructus aurantii with the mass ratio of 2-5:1-10 in the extracting tank for extraction with water, and obtaining an extracted fluid after extraction is finished, wherein the steam pressure of extraction is 0.25-0.35MPa, and the temperature is 70-90°C; S2, performing vacuum concentration on the extracted fluid to obtain a extract, wherein the vacuum degree of vacuum concentration is -0.08 to -0.06MPa, the steam pressure of extraction is 0.25-0.35MPa, and the temperature is 60-70°C; and S3, preparing the traditional Chinese medicine composition for loosening bowel to relieve constipation with the extract as the active ingredient. Preferably, extraction is performed two or more times. More preferably, extraction includes: adding 6-10 times water for the first time, and performing extraction for 4h; adding 4-8 times water for the second time, and performing extraction for 3h; and combining the first extracted fluid obtained by the first extraction with the second extracted fluid obtained by the second extraction, and filtering the mixture to obtain the extracted fluid. Further preferably, S3 further includes the following steps of drying and crushing the extract to obtain the dry paste powder, and then preparing the traditional Chinese medicine composition for loosening bowel to relieve constipation with the dry paste powder as the active ingredient.

Although all the compositions and contents thereof in the extract obtained by the steps cannot be specified, the inventor discovers that the traditional Chinese medicine composition obtained by the steps has relatively good curative effect, and meanwhile, the risk capable of producing the side effect is greatly lowered due to simple components of original raw materials.

According to a typical embodiment of the present disclosure, use of the traditional Chinese medicine composition for loosening bowel to relieve constipation in preparing a drug for loosening bowel to relieve constipation is provided.

The beneficial effect of the present disclosure will be further described below in combination with the embodiments.

Embodiment 1

I. Influence of sample 1# on small intestinal peristalsis of a mouse

1. Principle

Compound diphenoxylate as a model construction drug is administrated by oral gavage, a mouse small intestinal peristalsis inhibition model is established, the intestinal ink impelling ratio in a certain time is calculated, and the gastrointestinal peristalsis function of the model mouse is judged.

2. Sample and instruments

2.1. Sample and instruments

2.1.1 Sample 1#: provided by Tsing Hua De Ren Xi 'an Happiness Pharmaceutical Co., LTD (the aloe and the fructus aurantii with a ratio of 5:7 are added, boiling and extraction are performed with a proper quantity of water, and concentration is performed until a volume of 500ml to obtain a product, called as a stock solution), and each 1ml of the sample 1# is equivalent to 0.4152g of a total crude drug amount.
A preparation method of the stock solution includes the following steps of:
putting the aloe and the fructus aurantii in a multifunctional extracting tank at the same time; adding 6-10 times water for the first time, performing extraction for 4h, adding 4-8 times water for the second time, and performing extraction for 3h, wherein steam pressure is 0.25-0.35MPa, and a temperature is 80°C; filtering, combining filtrates, and performing vacuum concentration on a product, wherein a vacuum degree of vacuum concentration is -0.08 to -0.06MPa, steam pressure is 0.25-0.35MPa, and a temperature is 60-70°C; and performing concentration until a volume of 500ml to obtain a product, called as a stock solution.
2.1.2 Instruments: surgical scissors, ophthalmic forceps, a ruler, a syringe, activated carbon powder, acacia gum, compound diphenoxylate tablets, a measuring cylinder, distilled water, a tray and a timer.

2.2 Reagent preparation

2.2.1 Preparation of sample 1#

Low dose of 0.24ml/20ml/kg, distilled water is added in 0.24ml of the stock solution until a volume of 20ml.
Middle dose of 2.40ml/20ml/kg, distilled water is added in 2.40ml of the stock solution until a volume of 20ml.
High dose of 7.20ml/20ml/kg, distilled water is added in 7.20ml of the stock solution until a volume of 20ml.
Administration volumes of the mouse are all 20ml/kg, once a day.

2.2.2 Preparation of ink

50g of acacia gum is accurately weighed, 400ml of water is added, boiling is performed until a solution is transparent, 25g of activated carbon (powdery) is weighed and added in the solution for boiling three times, the water is added in the solution to dilute the solution to 500ml after the solution is cool, the solution is preserved in a refrigerator with a temperature of 4°C, and the solution is evenly shaken before being used.

2.2.3 Preparation of compound diphenoxylate with a concentration being 0.025%

For compound diphenoxylate tablets, each tablet contains 2.5mg of the compound diphenoxylate, 25mg of compound diphenoxylate tablets (10 pieces) are taken, are ground by a mortar into powder and is added with water to 100ml, and the compound diphenoxylate is prepared before using.

3. Experimental method

3.1 Experimental animal

60 Kunming male mice weighing 18-22g were purchased from Animal Experiment Center of Xi'an Jiaotong University.

3.2 Experimental steps

3.2.1 Grouping and administration of experiment animals

The mice were randomly divided into blank control group, model control group, sample 1# low-dose group, sample 1# medium-dose group, and sample 1# high-dose group. There were 5 groups in total, with 12 mice in each group. The blank control group and the model control group are given distilled water by oral gavage and are given a test sample by a same route with an administration volume being 20ml/Kg, once a day, for 10 days.

3.2.2 Establishment of model

The mice in each group are deprived of food but not water for 16h after intragastric administration for 10 days. The model control group and each dose group of the samples are given the compound diphenoxylate (5mg/kg BW) by oral gavage, and the blank control group is given the distilled water.

3.2.3 Specific method of index determination

After the compound diphenoxylate solution was administered for 0.5h, each dose group was by oral gavage given the ink containing the corresponding sample (containing 5% activated carbon powder and 10% gum arabic), and the blank control group and the model control group were given ink.by oral gavage The mice are sacrificed by cervical dislocation immediately after 25 minutes, abdominal cavities are opened to separate mesentery, intestines with the tops starting from pylori and the bottoms reaching ileocecus are scissored and are put on the tray, each small intestine is stretched into a straight line, the intestine length is measured as a "small intestine total length", and a length between the pylorus and the frontier of ink is "an ink impelling length". The ink impelling ratio (%) is calculated according to a following equation:

$$\text{Ink impelling ratio(\%)}=(\text{ink impelling length(cm)/small intestine total length(cm)})*100\%$$

4. Data processing

Data can be analyzed by using a variance, statistics is performed by a pairwise comparison method among the means of multiple experimental groups and one control group, and details are shown in Table 1 below.

Table 1 Influence Of Sample 1# On Mouse Small Intestinal peristalsis ($\bar{x}\pm$s n=12)

| Group | Dose/ml·kg$^{-1}$ | Ink Impelling Ratio (%) | P Value |
|---|---|---|---|
| Blank Control Group | - | 29.8$\pm$7.3 | |
| Model Control Group | - | 19.8$\pm$9.7$\triangle$ | 0.009 |
| Low-Dose Group | 0.24 | 28.8$\pm$11.1* | 0.046 |
| Middle-Dose Group | 2.40 | 29.0$\pm$9.23* | 0.026 |
| High-Dose Group | 7.20 | 30.6$\pm$5.13** | 0.002 |

Note: compared with the blank control group, $\triangle$P<0.01; compared with the model control group, **P<0.01; and compared with the model control group, *P<0.05.

5. Experimental result

Compared with the blank control group, the ink impelling ratio of the model control group has a highly statistically significant (P<0.01), which states that the model was established. Compared with the model control group, the ink impelling ratio of each dose group of the sample 1# has a highly statistically significant (P<0.05 or P<0.01).

II. Influence of sample 1# on defecation of a mouse

1. Principle

Compound diphenoxylate as the model construction drug is administrated by oral gavage, a mouse constipation model is established, and a first melena discharge time as well as a quantity and a weight of melena in 6h of the mouse are determined to reflect defecation of the model mouse.

2. Sample and instruments

2.1. Sample and instruments

2.1.1 Sample 1#: provided by Tsing Hua De Ren Xi 'an Happiness Pharmaceutical Co., LTD (the aloe and the fructus aurantii with a ratio of 5:7 are added, boiling and extraction are performed with a proper quantity of water, and concentration is performed until a volume of 500ml), and each 1ml of the sample 1# is equivalent to 0.4152g of a total crude drug.
A preparation method of the stock solution includes the following steps of:
putting the aloe and the fructus aurantii in a functional extracting tank at the same time; adding 6-10 times water for the first time, performing extraction for 4h, adding 4-8 times water for the second time, and performing extraction for 3h, wherein steam pressure is 0.25-0.35MPa, and a temperature is 80°C; filtering, combining filtrates, and performing vacuum concentration on a product, wherein a vacuum degree of vacuum concentration is -0.08 to -0.06MPa, steam pressure is 0.25-0.35MPa, and a temperature is 60-70°C; and performing concentration until a volume of 500ml to obtain a product, called as a stock solution.
2.1.2 Instruments: surgical scissors, ophthalmic forceps, a ruler, a syringe, activated carbon powder, acacia gum, compound diphenoxylate tablets, a measuring cylinder, distilled water, a tray and a timer.

2.2 Reagent preparation

2.2.1 Preparation of sample 1#

Low dose of 0.24ml/20ml/kg, distilled water is added in 0.24ml of the stock solution until a volume of 20ml.
Middle dose of 2.40ml/20ml/kg, distilled water is added in 2.40ml of the stock solution until a volume of 20ml.
High dose of 7.20ml/20ml/kg, distilled water is added in 7.20ml of the stock solution until a volume of 20ml.
Administration volumes of the mouse are all 20ml/kg, once a day.

2.2.2 Preparation of ink

50g of acacia gum is accurately weighed, 400ml of water is added, boiling is performed until a solution is transparent, 25g of activated carbon (powdery) is weighed and added in the solution for boiling three times, the water is added in the solution to dilute the solution to 500ml after the solution is cool, the solution is preserved in a refrigerator with a temperature of 4°C, and the solution is evenly shaken before being used.
2.2.3 Preparation of compound diphenoxylate solution with a concentration being 0.05%
For compound diphenoxylate tablets, each tablet contains 2.5mg of the compound diphenoxylate, 50mg of compound diphenoxylate tablets (20 pieces) are taken, are ground by a mortar into powder and is added with distilled water to 100ml, and the compound diphenoxylate solution is prepared before using.

3. Experimental method

3.1 Experimental animal

60 Kunming male mice weighing 18-22g were purchased from Animal Experiment Center of Xi'an Jiaotong University.

3.2 Experimental steps

3.2.1 Grouping and administration of experiment animals

The mice were randomly divided into blank control group, model control group, sample 1# low-dose group, sample 1# medium-dose group, and sample 1# high-dose group. There were 5 groups in total, with 12 mice in each group. The blank control group and the model control group are given distilled water by oral gavage and are given a test sample by a same route with an administration volume being 20ml/Kg, once a day, for 10 days.

### 3.2.2 Establishment of model

The mice in each group are deprived of food but not water for 16h after intragastric administration for 10 days. The model control group and each dose group of the sample 1# are given the compound diphenoxylate solution (5mg/kg BW) by oral gavage, and the blank control group is given the distilled water.

### 3.2.3 Specific method of index determination

After administration with the compound diphenoxylate solution for 0.5h, the mice in a negative control group and the model control group are given ink by oral gavage, the mice in the dose groups are given ink containing the test sample, and all animals are fed separately and eat normally.

Beginning from ink filling, the first melena discharge time as well as the quantity and the weight of the melena in 6h of each mouse are recorded.

### 4. Data processing and result determination

Data can be analyzed by using a variance, statistics is performed by a pairwise comparison method among the means of multiple experimental groups and one control group, and details are shown in Table 2, Table 3 and Table 4 below.

Table 2 Influence of Sample 1# On Mouse First Melena Discharge Time ($\bar{x}\pm$s n=12)

| Group | Dose/ml·kg$^{-1}$ | Melena Discharge Time (S) | P Value |
|---|---|---|---|
| Blank Control Group | - | 61.8±35.7 | |
| Model Control Group | - | 166.5±73.9Δ | 0.0002 |
| Low-Dose Group | 0.24 | 159.6±46.1 | 0.786 |
| Middle-Dose Group | 2.40 | 157.8±44.3 | 0.728 |
| High-Dose Group | 7.20 | 144.1±37.2 | 0.358 |

Note: compared with the blank control group, ΔP<0.01; compared with the model control group, **P<0.01; and compared with the model control group, *P<0.05.

Compared with the blank control group, the first melena discharge time of the model control group has a highly statistically significant (P<0.01), which states that the model was established. Compared with the model control group, the first melena discharge time of each dose group of the sample 1# is shortened without a statistic significant (P>0.05).

Table 3 Influence of Sample 1# On Mouse Melena Quantity ($\bar{x}\pm$s n=12)

| Group | Dose/ml.kg$^{-1}$ | Melena Quantity (Grain) | P Value |
|---|---|---|---|
| Blank Control Group | - | 34.9±6.52 | |
| Model Control Group | - | 27.4±10.5Δ | 0.047 |
| Low-Dose Group | 0.24 | 37.3±12.0* | 0.042 |
| Middle-Dose Group | 2.40 | 37.3±9.08* | 0.023 |
| High-Dose Group | 7.20 | 36.2±9.26* | 0.042 |

Note: compared with the blank control group, ΔP<0.05; compared with the model control group, **P<0.01; and compared with the model control group, *P<0.05.

Compared with the blank control group, the melena quantity of the model control group has a highly statistically significant (P<0.05), which states that the model is prepared successfully. Compared with the model control group, each dose group of the sample 1# has a highly statistically significant (P<0.05).

Table 4 Influence of Sample 1# On Mouse Melena Weight ($\bar{x} \pm s$ n=12)

| Group | Dose/ml.kg$^{-1}$ | Melena Weight (g) | P Value |
|---|---|---|---|
| Blank Control Group | - | 1.20±0.36 | |
| Model Control Group | - | 1.26±0.59 | 0.761 |
| Low-Dose Group | 0.24 | 1.81±0.55* | 0.029 |
| Middle-Dose Group | 2.40 | 1.78±0.54* | 0.035 |
| High-Dose Group | 7.20 | 1.82±0.50* | 0.020 |

Note: compared with the blank control group, $\triangle P<0.01$; compared with the model control group, $**P<0.01$; and compared with the model control group, $*P<0.05$.

Compared with the blank control group, the melena weights of the model control group are increased without a statistical significance (P>0.05); and compared with the model control group, the melena weights of each dose group of the sample 1# are extremely statistically significant (P<0.05).

5. Result determination:

According to the situation that any result of a small intestinal peristalsis experiment and a defecations time is positive and the situation that any result of the quantity and the weight of the melena in 6h is positive, an experimental result can be determined to be positive.
The result shows that compared with the model control group, the ink impelling ratio of each dose group of the sample 1# has a statistically or highly statistically significant (P<0.05 or P<0.01); and the melena quantity of each dose group of the sample 1# has a highly statistically significant (P<0.05).

6. Conclusion

The effect of the sample 1# on small intestinal peristalsis and defecation of the mice is statistically significant or extremely statistically significant (P<0.05 or P< 0.01).

Embodiment 2

Influence of traditional Chinese medicine composition with different ratios on effect of loosening bowel to relieve constipation

1. Principle: the beneficial effects of the ratio of the traditional Chinese medicine composition (the aloe and the fructus aurantii) of the present disclosure are found by a mouse intestinal peristalsis experiment result and a mouse defecation experiment result.

1.1 Mouse intestinal peristalsis experiment: compound diphenoxylate as a model construction drug is administrated by oral gavage, a mouse small intestinal peristalsis inhibition model is established, the intestinal ink impelling ratio in a certain time is calculated, and the gastrointestinal peristalsis function of the model mouse is judged.
1.2 Mouse defecation experiment: the compound diphenoxylate as the model construction drug is administrated by oral gavage, a mouse constipation model is established, and a first melena discharge time as well as a quantity and a weight of melena in 6h of the mouse are determined to reflect defecation of the model mouse.

2. Sample and instruments

2.1. Sample and instruments

2.1.1 Sample source: the sample is provided by Tsing Hua De Ren Xi 'an Happiness Pharmaceutical Co., LTD.

2.1.2 Sample preparation

(1) Ratio I sample: the aloe and the fructus aurantii with a ratio of 5 to 1 are added, boiling and extraction are performed with a proper quantity of water, and concentration is performed until a volume of 500ml to obtain a product, called as a stock solution I. Every 1ml of the stock solution I is equivalent to 0.4152g of the total crude drug amount.
(2) Ratio II sample: the aloe and the fructus aurantii with a ratio of 5:7 are added, boiling and extraction are performed with a proper quantity of water, and concentration is performed until a volume of 500ml to obtain a product, called as a stock solution II. Every 1ml of the stock solution I is equivalent to 0.4152g of the total crude drug amount.
(3) Ratio III sample: the aloe and the fructus aurantii with a ratio of 1 to 5 are added, boiling and extraction are performed with a proper quantity of water, and concentration is performed until a volume of 500ml to obtain a product, called as a stock solution III. Every 1ml of the stock solution I is equivalent to 0.4152g of the total crude drug amount.

2.1.2 Instruments: surgical scissors, ophthalmic forceps, a ruler, a syringe, activated carbon powder, acacia gum, compound diphenoxylate tablets, a measuring cylinder, distilled water, a tray and a timer.

2.2 Reagent preparation

2.2.11 Administration dose setting

The stock solution I, the stock solution II and the stock solution III are prepared according to a low dose, a middle dose and a high dose, and methods are as follows:

(1) Low dose: 0.24ml/20ml/kg, distilled water is added in 0.24ml of the stock solution until a volume of 20ml.
(2) Middle dose: 2.40ml/20ml/kg, distilled water is added in 2.40ml of the stock solution until a volume of 20ml.
(3) High dose: 7.20ml/20ml/kg, distilled water is added in 7.20ml of the stock solution until a volume of 20ml.

Administration volumes of the mouse are all 20ml/kg, once a day.

2.2.2 Preparation of ink

50g of acacia gum is accurately weighed, 400ml of water is added, boiling is performed until a solution is transparent, 25g of activated carbon (powdery) is weighed and added in the solution for boiling three times, the water is added in the solution to dilute the solution to 500ml after the solution is cool, the solution is preserved in a refrigerator with a temperature of 4°C, and the solution is evenly shaken before being used.

2.2.3 Preparation of compound diphenoxylate solution

(1) 0.025% compound diphenoxylate solution: 10 compound diphenoxylate tablets (each of which contains 2.5mg of the compound diphenoxylate) are taken, are ground by a mortar into powder and is added with distilled water to 100ml, and the 0.025% compound diphenoxylate solution is prepared before using.
(2) 0.05% compound diphenoxylate solution: 20 compound diphenoxylate tablets (each of which contains 2.5mg of the compound diphenoxylate) are taken, are ground by a mortar into powder and are added with distilled water to 100ml, and the 0.05% compound diphenoxylate solution is prepared before using.

3. Mouse intestinal peristalsis experiment:

3.1. Experimental method

3.1.1 Experimental animal

132 Kunming male mice weighing 18-22g were purchased from Animal Experiment Center of Xi'an Jiaotong University.

3.1.2 Experimental steps

3.1.2.1 Grouping and administration of experiment animals

The mice were randomly divided into blank control group, model control group, low-dose group of a ratio I sample, middle-dose group of the ratio I sample, high-dose group of the ratio I sample, low-dose group of a ratio II sample, middle-dose group of the ratio II sample, high-dose group of the ratio II sample, low-dose group of a ratio III sample, middle-dose group of the ratio III sample and high-dose group of the ratio III sample by weight. There were 11 groups in total, with 12 mice in each group. The blank control group and the model control group are given distilled water by oral gavage and are given a test sample by a same route with an administration volume being 20ml/Kg, once a day, for 10 days.

3.1.2.2 Establishment of model

The mice in each group are deprived of food but not water for 16h after intragastric administration for 10 days. The model control group and each dose group of the samples are given the 0.025% compound diphenoxylate solution (5mg/kg BW) by oral gavage, and the blank control group is given the distilled water.

3.1.2.3 Specific method of index determination

After the compound diphenoxylate solution was administered for 0.5h, each dose group was by oral gavage given the ink containing the corresponding sample (containing 5% activated carbon powder and 10% gum arabic), and the blank control group and the model control group were given ink.by oral gavage The mice are sacrificed by cervical dislocation immediately after 25 minutes, abdominal cavities are opened to separate mesentery, intestines with the tops starting from pylori and the bottoms reaching ileocecus are scissored and are put on the tray, each small intestine is stretched into a straight line, the intestine length is measured as a "small intestine total length", and a length between the pylorus and the frontier of ink is "an ink impelling length". The ink impelling ratio (%) is calculated according to a following equation:

$$\text{Ink impelling ratio(\%)} = (\text{ink impelling length(cm)/small intestine total length(cm)}) * 100\%$$

3.2. Data processing

Data is analyzed by using a variance, statistics is performed by a pairwise comparison method among the means of multiple experimental groups and one control group, and details are shown in Table 5 below.

Table 5 Influence of Traditional Chinese Medicine Composition with Different Ratios on Mouse Small Intestinal peristalsis ($\bar{x} \pm s$ n=12)

| Group | | | Dose/ml.kg$^{-1}$ | Ink Impelling Ratio (%) | P Value |
|---|---|---|---|---|---|
| Blank Group | Control | | - | 29.8±7.26 | |
| Model Group | Control | | - | 19.8±9.73△ | 0.009 |
| Ratio I | | Low-Dose Group | 0.24 | 25.1±6.14 | 0.089 |
| | | Middle-Dose Group | 2.40 | 27.6±6.47* | 0.045 |
| | | High-Dose Group | 7.20 | 29.2±7.52* | 0.021 |
| Ratio II | | Low-Dose Group | 0.24 | 28.8±11.1* | 0.046 |
| | | Middle-Dose Group | 2.40 | 29.0±9.23* | 0.026 |

(continued)

| Group | | Dose/ml.kg$^{-1}$ | Ink Impelling Ratio (%) | P Value |
|---|---|---|---|---|
| Ratio III | High-Dose Group | 7.20 | 30.6±5.13** | 0.002 |
| | Low-Dose Group | 0.24 | 24.8±8.12 | 0.103 |
| | Middle-Dose Group | 2.40 | 27.4±5.96* | 0.049 |
| | High-Dose Group | 7.20 | 28.1±7.30* | 0.048 |

Note: compared with the blank control group, ΔP<0.01; compared with the model control group, **P<0.01; and compared with the model control group, *P<0.05.

3.3 Experimental result

Compared with the blank control group, the ink impelling length of the model control group is extremely statistically significant (P<0.01), which states that the model was established. Compared with the model control group, the ink impelling ratio of each dose group of the sample is increased, and the ink impelling ratios of the middle-dose group of the ratio I sample, the high-dose group of the ratio I sample, the low-dose group of the ratio II sample, the middle-dose group of the ratio II sample, the high-dose group of the ratio II sample, the middle-dose group of the ratio III sample and the high-dose group of the ratio III sample are extremely statistically significant (P<0.05).

4. Mouse Defecation Experiment

4.1. Experimental method

4.1.1 Experimental animal

132 Kunming male mice weighting 18-22g were purchased from Animal Experiment Center of Xi'an Jiaotong University.

4.1.2 Experimental steps

4.1.2.1 Grouping and administration of experiment animals

The mice were randomly divided into blank control group, model control group, low-dose group of a ratio I sample, middle-dose group of the ratio I sample, high-dose group of the ratio I sample, low-dose group of a ratio II sample, middle-dose group of the ratio II sample, high-dose group of the ratio II sample, low-dose group of a ratio III sample, middle-dose group of the ratio III sample and high-dose group of the ratio III sample by weight. There were 11 groups in total, with 12 mice in each group. The blank control group and the model control group are given distilled water by oral gavage and are given a test sample by a same route with an administration volume being 20ml/Kg, once a day, for 10 days.

4.1.2.2 Establishment of model

The mice in each group are deprived of food but not water for 16h after intragastric administration for10 days. The model control group and each dose group of the samples are given the 0.05% compound diphenoxylate solution (5mg/kg BW) by oral gavage, and the blank control group is given the distilled water.

4.1.2.3 Specific method of index determination

After administration with the compound diphenoxylate solution for 0.5h, the mice in the blank control group and the model control group are given ink by oral gavage, the mice in the dose groups are given ink containing the test sample, and all animals are feed separately and eat normally.
Beginning from ink filling, a first melena discharge time as well as a quantity and a weight of the melena in 6h of each mouse are recorded.

4.2. Data processing and result determination

Data can be analyzed by using a variance, statistics is performed by a pairwise comparison method among the means of multiple experimental groups and one control group, and details are shown in Table 6, Table 7 and Table 8 below.

Table 6 Influence of Traditional Chinese Medicine Composition with Different Ratios on Mouse First Melena Discharge Time ($\bar{x}\pm s$ n=12)

| Group | | | Dose/ml.kg$^{-1}$ | Melena Time (S) | Discharge | P Value |
|---|---|---|---|---|---|---|
| Blank Group | Control | | - | 61.8.8±35.7 | | |
| Model Group | Control | | - | 166.5±73.9▲ | | 0.0002 |
| Ratio I | | Low-Dose Group | 0.24 | 161.8±45.4 | | 0.792 |
| | | Middle-Dose Group | 2.40 | 158.1±46.3 | | 0.734 |
| | | High-Dose Group | 7.20 | 146.7±39.4 | | 0.379 |
| Ratio II | | Low-Dose Group | 0.24 | 159.6±46.1 | | 0.786 |
| | | Middle-Dose Group | 2.40 | 157.8±44.3 | | 0.728 |
| | | High-Dose | 7.20 | 144.1±37.2 | | 0.358 |
| Ratio III | | Group Low-Dose Group | 0.24 | 162.3±48.2 | | 0.798 |
| | | Middle-Dose Group | 2.40 | 159.6±51.2 | | 0.749 |
| | | High-Dose Group | 7.20 | 147.4±42.7 | | 0.387 |

Note: compared with the blank control group, $\triangle$P<0.01; compared with the model control group, **P<0.01; and compared with the model control group, *P<0.05.

Compared with the blank control group, the first melena discharge time of the model control group is extremely statistically significant (P<0.01), which states that the model was established. Compared with the model control group, the first melena discharge time of the middle-dose groups and the high-dose groups of the ratio I sample, the ratio II sample and the ratio III sample are shortened without a statistical significance.

Table 7 Influence of Traditional Chinese Medicine Composition with Different Ratios on Mouse Melena Quantity ($\bar{x}\pm s$, n=12)

| Group | | | Dose/ml·kg$^{-1}$ | Melena Quantity (Grain) | P Value |
|---|---|---|---|---|---|
| Blank Group | Control | | - | 34.9±6.52 | |
| Model Group | Control | | - | 27.4±10.5$\triangle$ | 0.047 |
| Ratio I | | Low-Dose Group | 0.24 | 37.8±13.3 | 0.057 |
| | | Middle-Dose Group | 2.40 | 36.5±9.87* | 0.047 |
| | | High-Dose Group | 7.20 | 36.3±8.43* | 0.035 |
| Ratio II | | Low-Dose Group | 0.24 | 37.3±12.0* | 0.042 |
| | | Middle-Dose Group | 2.40 | 37.3±9.08* | 0.023 |

(continued)

| Group | | Dose/ml·kg⁻¹ | Melena Quantity (Grain) | P Value |
|---|---|---|---|---|
| | High-Dose Group | 7.20 | 36.2±9.26* | 0.042 |
| Ratio III | Low-Dose Group | 0.24 | 36.7±10.9 | 0.051 |
| | Middle-Dose Group | 2.40 | 37.4±10.0* | 0.022 |
| | High-Dose Group | 7.20 | 36.9±11.6* | 0.045 |

Note: compared with the blank control group, △P<0.05; compared with the model group, **P<0.01; and compared with the model group, *P<0.05.

Compared with the blank control group, the melena quantity of the model control group is extremely statistically significant (P<0.05), which states that the model is prepared successfully. Compared with the model control group, the melena quantity of each dose group of the ratio I sample, the ratio II sample and the ratio III sample are obviously increased, and the middle-dose groups and the high-dose groups are extremely statistically significant (P<0.05).

Table 8 Influence of Traditional Chinese Medicine Composition with Different Ratios on Mouse Melena Weight ($\bar{x}\pm s$ n=12)

| Group | | | Dose/ml·kg⁻¹ | Melena (g) | Weight | P Value |
|---|---|---|---|---|---|---|
| Blank Group | Control | | - | 1.20±0.36 | | |
| Model Group | Control | | - | 1.26±0.59 | | 0.761 |
| Ratio I | | Low-Dose Group | 0.24 | 1.77±0.68* | | 0.037 |
| | | Middle-Dose Group | 2.40 | 1.79±0.71* | | 0.032 |
| | | High-Dose Group | 7.20 | 1.82±0.65* | | 0.020 |
| Ratio II | | Low-Dose Group | 0.24 | 1.81±0.55* | | 0.029 |
| | | Middle-Dose Group | 2.40 | 1.78±0.54* | | 0.035 |
| | | High-Dose Group | 7.20 | 1.82±0.50* | | 0.020 |
| Ratio III | | Low-Dose Group | 0.24 | 1.75±0.43* | | 0.039 |
| | | Middle-Dose Group | 2.40 | 1.78±0.55* | | 0.035 |
| | | High-Dose Group | 7.20 | 1.80±0.50* | | 0.033 |

Note: compared with the blank control group, △P<0.01; compared with the model control group, **P<0.01; and compared with the model control group, *P<0.05.

Compared with the blank control group, the melena weight of the model control group is increased without a statistical significance (P>0.05). Compared with the model control group, the melena weight of each dose group of the ratio I sample, the ratio II sample and the ratio III sample are extremely statistically significant (P<0.05).

4.3 Experimental result

According to the situation that any result of a small intestinal peristalsis experiment and a defecations time is positive, currently with that any result of the quantity and the weight of the melena in 6h is positive, an experimental result can be determined to be positive.

The result shows that compared with the model control group, the ink impelling ratio of each dose group of the ratio I sample, the ratio II sample and the ratio III sample are significant or extremely statistically significant ($P<0.05$ or $P<0.01$); and the melena quantity of each dose group of the ratio I sample, the ratio II sample and the ratio III sample are extremely statistically significant ($P<0.05$).

5. Conclusion

Through the mouse intestinal peristalsis experiment and the mouse defecation experiment, it can be known that the samples of the ratio I (5:1), the ratio II (5:7) and the ratio III (1:5) all have certain effect of loosening bowel to relieve constipation, and the effect strength of loosening bowel to relieve constipation is as follows: the ratio II (5:7) >the ratio I (3:1)>the ratio III (1:5).

Furthermore, considering the safety problem of an aloe-fructus aurantii composition, a proportion of the aloe should be properly lowered, and therefore, the ratio II (5:7) is the optimal ratio.

Embodiment 3

Comparative advantage of traditional Chinese medicine composition related to effect of loosening bowel to relieve constipation

1. The aloe-fructus aurantii traditional Chinese medicine composition (the sample 1# in Embodiment 1) and an aloe-radix angelicae sinensis-radix ophiopogonis-fructus aurantii traditional Chinese medicine composition (a preparation method of which refer to patent: a traditional Chinese medicine composition for loosening bowel to relieve constipation and preparation of the traditional Chinese medicine composition with a Patent Number being ZL201110317780.9) are proved by mouse function experiments that the middle-dose group and the high-dose group of each composition both have the efficacy of loosening bowel to relieve constipation. Computational statistics is performed on mice (calculated as 20g) daily crude drug intake amount of the sample of each group (seeing Table 9), and known from the result that on the premise of exerting the effect of loosening bowel to relieve constipation, the mouse daily crude drug intake amount in an aloe-fructus aurantii group is lower than that in an aloe-radix angelicae sinensis-radix ophiopogonis-fructus aurantii group.

Table 9 Mouse Daily Crude Drug Intake Amount (g)

| Group | Aloe-fructus aurantii | Aloe-radix angelicae sinensis-radix ophiopogonis-fructus aurantii |
|---|---|---|
| Middle-Dose Group | 0.01992 | 0.0436 |
| High-Dose Group | 0.05976 | 0.0872 |

2. A proportion of the aloe in the aloe-fructus aurantii traditional Chinese medicine composition is 41.7%, a proportion of the aloe in the aloe-radix angelicae sinensis-radix ophiopogonis-fructus aurantii traditional Chinese medicine composition is 50.9%, and the use ratio of the aloe is obviously reduced, such that the safety of each composition is improved, and the risk of producing the side effect after each composition is taken is lowered.

What stated above are merely preferred embodiments of the present disclosure but are not used to limit the present disclosure, and various modifications and variations can be made in the present disclosure to those skilled in the art. Any modifications, equivalent substitutions, improvements and the like within the spirit and principles of the present disclosure are intended to be embraced by the protection range of the present disclosure.

**Claims**

1. A traditional Chinese medicine composition for loosening bowel to relieve constipation, wherein active ingredients of the traditional Chinese medicine composition are prepared from extracts of raw materials which consist of aloe and fructus aurantii with a mass ratio of 2-5:1-10.

2. The traditional Chinese medicine composition according to claim 1, wherein the raw materials consist of the aloe and the fructus aurantii with a mass ratio of 5:7.

3. The traditional Chinese medicine composition according to claim 1, wherein the traditional Chinese medicine composition further comprises a pharmaceutically acceptable adjuvant.

**4.** The traditional Chinese medicine composition according to claim 3, wherein the traditional Chinese medicine composition is in the following dosage forms: tablets, granules, capsules, pills, suppositories, powders, concentrated decoction, drops, aerosol, powder for inhalation, solution, a suspension, syrup, mixture, medicinal wine, medicinal tea, buccal tablets, freeze-dried powder injection or an emulsion.

**5.** The traditional Chinese medicine composition according to any one of claims 1-4, wherein the traditional Chinese medicine composition is prepared by the following steps of:

S1, putting the aloe and the fructus aurantii with the mass ratio of 2-5:1-10 in an extracting tank for extraction with water, and obtaining an extracted fluid after extraction is finished, wherein steam pressure of extraction is 0.25-0.35MPa, and a temperature is 70-90°C;

S2, performing vacuum concentration on the extracted fluid to obtain a extract, wherein a vacuum degree of vacuum concentration is -0.08 to -0.06MPa, steam pressure of extraction is 0.25-0.35MPa, and a temperature is 60-70°C;

and S3, preparing the traditional Chinese medicine composition for loosening bowel to relieve constipation with the extract as the active ingredient.

**6.** The traditional Chinese medicine composition according to claim 5, wherein extraction is performed two or more times.

**7.** The traditional Chinese medicine composition according to claim 5, wherein extraction comprises:

adding 6-10 times water for the first time, and performing extraction for 4h;

adding 4-8 times water for the second time, and performing extraction for 3h;

and combining a first extracted fluid obtained by the first extraction with a second extracted fluid obtained by the second extraction, and filtering a mixture to obtain the extracted fluid.

**8.** The traditional Chinese medicine composition according to claim 5, wherein S3 further comprises the following steps of drying and crushing the extract to obtain dry paste powder, and then preparing the traditional Chinese medicine composition for loosening bowel to relieve constipation with the dry paste powder as the active ingredient.

**9.** A preparation method of the traditional Chinese medicine composition of any one of claims 1-8, comprising the following steps of:

S1, putting the aloe and the fructus aurantii with the mass ratio of 2-5:1-10 in an extracting tank for extraction with water, and obtaining an extracted fluid after extraction is finished, wherein the steam pressure of extraction is 0.25-0.35MPa, and the temperature is 70-90°C;

S2, performing vacuum concentration on the extracted fluid to obtain a extract, wherein the vacuum degree of vacuum concentration is -0.08 to -0.06MPa, the steam pressure of extraction is 0.25-0.35MPa, and the temperature is 60-70°C;

and S3, preparing the traditional Chinese medicine composition for loosening bowel to relieve constipation with the extract as the active ingredient;

preferably, extraction is performed two or more times;

more preferably, extraction comprises: adding 6-10 times water for the first time, and performing extraction for 4h; adding 4-8 times water for the second time, and performing extraction for 3h; and combining the first extracted fluid obtained by the first extraction with the second extracted fluid obtained by the second extraction, and filtering the mixture to obtain the extracted fluid;

and further preferably, S3 further comprises the following steps of drying and crushing the extract to obtain the dry paste powder, and then preparing the traditional Chinese medicine composition for loosening bowel to relieve constipation with the dry paste powder as the active ingredient.

**10.** Use of the traditional Chinese medicine composition of any one of claims 1-8 for loosening bowel to relieve constipation in preparing a drug for loosening bowel to relieve constipation.

## INTERNATIONAL SEARCH REPORT

| | |
|---|---|
| International application No. | |
| | **PCT/CN2020/077631** |

**A. CLASSIFICATION OF SUBJECT MATTER**

A61K 36/896(2006.01)i; A61K 36/886(2006.01)i; A61P 1/10(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNMED; DWPI; SIPOABS; CJFD; CNKI; MEDNPL: 芦荟, 枳壳, 便秘, 通便, Aloe?, Bitter Orange, Auranti??, constipation, coprostasis, astriction, emplastic, laxative?, deobstruent, cathartic, defecation, purgative

**C. DOCUMENTS CONSIDERED TO BE RELEVANT**

| Category* | Citation of document, with indication, where appropriate, of the relevant passages | Relevant to claim No. |
|---|---|---|
| Y | 孙培等 (SUN, Pei et al.). "高效液相色谱法测定芦荟胶囊中芦荟苷的含量 (Non-official translation: Determination of Aloin in LuHui Capsules by HPLC)" 《湖北中医药大学学报》 *(Journal of Hubei University of Traditional Chinese Medicine)*, Vol. 14, No. 2, 30 April 2012 (2012-04-30), pp. 43 and 44 | 1-10 |
| Y | 官扬等 (GUAN, Yang et al.）. "江枳壳润肠颗粒改善功能性便秘的研究 (Non-official translation: The Study of Citrus Aurantium L.particles to Improve the Intestinal Function in Functional Constipation)" 中药药理与临床 *(Pharmacology and Clinics of Chinese Materia Medica)*, Vol. 33, No. 1, 28 February 2017 (2017-02-28), pp. 148-150 | 1-10 |
| PX | CN 109939167 A (QINGHUA DEREN XI'AN XINGFU PHARMACEUTICAL LTD.) 28 June 2019 (2019-06-28) claims 1-10 | 1-10 |
| A | CN 102362970 A (QINGHUA DEREN XI'AN XINGFU PHARMACEUTICAL LTD.) 29 February 2012 (2012-02-29) entire document | 1-10 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 May 2020** | **09 June 2020** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2020/077631**

| Patent document cited in search report | | | Publication date (day/month/year) | Patent family member(s) | | | Publication date (day/month/year) |
|---|---|---|---|---|---|---|---|
| CN | 109939167 | A | 28 June 2019 | None | | | |
| CN | 102362970 | A | 29 February 2012 | CN | 102362970 | B | 08 May 2013 |

Form PCT/ISA/210 (patent family annex) (January 2015)